# EUROPEAN PATENT SPECIFICATION

(11) **EP 1 149 129 B1**
(45) Date of publication and mention of the grant of the patent: **20.04.2005**
(21) Application number: 99956722.5
(22) Date of filing: 03.11.1999
(51) Int. Cl.: C08L 15/00, C08L 23/00, C08L 25/06, A61L 15/22, A61L 15/42, A61L 15/58, A61L 24/04, C09J 123/16, C09J 153/00, C09J 101/00

(54) **HYDROCOLLOID ADHESIVE COMPOSITIONS**
HYDROCOLLOIDE KLEBSTOFFZUSAMMENSETZUNGEN
COMPOSITIONS ADHESIVES HYDROCOLLOIDES

(30) Priority: 03.11.1998 US 106799 P
(43) Date of publication of application: 31.10.2001
(73) Proprietor: BRISTOL-MYERS SQUIBB COMPANY, Skillman, NJ 08558 (US)
(72) Inventor: FATTMAN, George, Mt. Laurel, NJ 08054 (US)
(74) Representative: Holmes, Miles Keeton
(86) International application number: PCT/US1999/025248
(87) International publication number: WO 2000/026293

(56) References cited:
- EP-A- 0 756 854
- EP-A1- 0 693 290
- US-A- 4 231 369
- US-A- 5 429 591
- US-A- 5 622 711

## Description

### BACKGROUND OF THE INVENTION

The present invention relates to pressure sensitive adhesive compositions and more particularly to pressure sensitive hydrocolloid adhesive compositions suited for use in ostomy, wound and incontinence care.

The compositions of this invention can be used for example to hold ostomy or incontinence devices onto the body of the user or to hold wound dressings in position for treating skin ailments or as the wound dressing itself.

Various hydrocolloid adhesive compositions suitable for medical uses are disclosed in the patent literature, e. g., U. S. Patent Nos. 3,339,549 (Chen); 4,192,785 (Chen, et al.); 4,166,051 (Cilento, et al.); 4,393,080 (Pawelchak, et al.); 3,612,053 (Pratt, et al.); 4,231,369 (Sorensen, et al.); 3,908,658 (Marsan); 4,367,732 (Poulsen, et al.); 4,378,018 (Alexander, et al.); and 4,393,150 (Krones).

Hydrocolloid adhesives are composed of powdered hydrocolloids dispersed in an elastomeric polymer matrix. They have been found to make excellent pressure sensitive adhesives for attachment of devices to the skin. Useful hydrocolloid adhesives depend on their initial tack, cohesive strength, and absorbency to achieve wearing times on skin of up to 10 days or longer. It is also required that components of hydrocolloid adhesives be cost effective, easy to process, and compatible with other device components.

EP 0 693 290 A1 describes the use of a matrix of an hydrocolloid dressing, which is constituted by an adhesive elastomeric network wherein one hydrocolloid compound is inserted, as a topical haemostatic agent.

EP 0 756 854 A1 describes an adhesive wafer having a barrier layer of soft adhesive material with particles of a hydrocolloid dispersed therein. Moreover a flexible cover layer extends along one side of the barrier layer and a sheet protects the opposite side of the barrier layer. The wafer has a central zone and a surrounding zone, wherein the material of the barrier layer in the outer zone has generally uniform molecular orientation in radial directions.

US 4 231 369 describes a sealing material for use in connection with ostomy devices to surround and protect a stoma in the human body. The material is a shaped gel-like composition composed of a continuous phase consisting of such elastomers, and preferably a hydrocarbon tackifier resins and likewise optionally an oil extender. One or more hydrocolloids are distributed in the continuous phase

### SUMMARY OF THE INVENTION

The present invention is defined in the claims.

An advantage of the present invention is that it can provide a biocompatible hydrocolloid adhesive composition simultaneously having preferred probe tack, tensile strength and saline absorbency as provided in Table 1.

**Table 1**

| Characteristic | Usefulness Range | Preferred Range | |
|---|---|---|---|
| Probe Tack | About 300-750 grams, force | About 500-650 grams, force | |
| Tensile Strength | About 500-3500 grams/cm² | About 1500-2500 g/cm² extruded | About 500-1500 non-extruded |
| Saline Absorbency | About 500-5000 g/m²/day | About 2000-3500 g/m²/d extruded | About 1500-2500 non extruded |

Another advantage of the present invention is that it can provide a biocompatible adhesive that is easy to process, cost efficient, durable and that doesn't swell excessively when hydrated.

Another advantage of the present invention is that it can achieve probe tack values above about 300 grams, force, as this value has been found to be desirable for attaining a strong initial bond to the skin. Improved bonds are generally obtained with increased tack, though too strong a bond may damage skin upon removal

Another advantage of the present invention is that it can achieve acceptable shear strength and peel properties. It has been determined that tensile strengths of about 500-3500 g/square centimeter are desirable. It is also recognized, however, that decreased flexibility or reduced adhesion may result if cohesive strength is excessive.

### DESCRIPTION OF THE PREFERED EMBODIMENTS

The adhesive compositions of the present invention utilize a poly (ethylene-propylene) rubber (EPR) having a broad molecular weight, amorphous structure and an ethylene content of 50% or less. The EPR is also a random copolymer and easy to process. The preferred EPR is Vistalon 404, Exxon Chemical Co. Low molecular weight species in the EPR favor bond formation while higher molecular weight species enhance cohesive and peel strength.

A compatible tackifying resin for this elastomer preferred for optimum tackification is a low molecular weight, highly hydrogenated polyvinylcyclohexane. Commercially available examples are Regalrez®) 1085 or Regalrez® 1094. (Hercules, Inc.). It is further preferred that the polyvinylcyclohexane have a softening point below body temperature and most preferably below room temperature such that it may further improve the wetting of skin by the elastomeric component. A commercially available example is Regalrez® 1018 (Hercules, Inc.). In particular, the use of low softening point resin allows for increasing the concentration of tackifier by a corresponding reduction in the concentration of plasticizing components. Both factors favorably influence adhesion, while retaining flexibility that may be lost when resins of higher softening point are employed. Adhesion is easily measured by using a task test to determine the bond forming capability.

Because hydrocolloid adhesives are composed of an elastomeric phase and a hydrocolloid phase, proper optimization of the adhesive formulation requires simultaneous optimization of the individual phases. While some interaction does occur between phases, the elastomeric phase is primarily responsible for the adhesive and cohesive strength properties, and the hydrocolloid phase is primarily responsible for the overall moisture handling and moisture interaction characteristics of the formulation. Adhesion and cohesion typically represent a balance of competing behaviors, and strong elastomers may not yield very effective adhesives. Moisture handling is more than just absorption, per se, but must be thought of in terms of absorption rate, absorption capacity, and the properties of the hydrated material. The hydrocolloid phase is key in determining these characteristics.

The ability of the adhesive to form a bond to the skin is directly related to the probe tack of the adhesive. Tack is generated by the action of tackifying resins to enhance wetting of the skin by the elastomeric components of the formulation. Modification of elastomers by tackifiers enables elastomers to form bonds while retaining their cohesiveness and contribute strength to the overall formulation. Compatibility of the tackifier with the elastomer is believed to be a key factor in tackifier effectiveness, as is the presence of low molecular weight species in the elastomer. Typically, the elastomer is blended with a styrenic block copolymer for optimum cohesive strength.

In order to attain adhesion to the skin for more than 1 or 2 days, it is necessary that the adhesive absorb fluid at a rate greater than or equal to the rate of transepidermal water loss (TEWL), approximately 7-10 g/square meter per hour on average. The actual absorption rate required to maintain skin adhesion can in practice be somewhat greater than this value as a function of weather, physical activity, etc. To obtain the minimum absorption capacity, this absorption rate should be multiplied over the wearing time.

Absorption by the adhesive is necessary to effectively manage perspiration from the skin. Without this capability, the adhesive would fail after short wearing times, and skin condition would be adversely affected. However, absorption also causes degradation of the adhesive component resulting from hydration and chemical attack by stomal discharge. This degradation occurs because the absorbent hydrocolloids act regardless of the source of the fluid absorbed, either perspiration or stoma effluent. Therefore, limiting absorption to the minimum necessary to manage perspiration will limit how stoma fluid will impact dressing integrity.

Denture adhesives are an example of water activated adhesives. Typically, sodium carboxy-methyl cellulose (NaCMC) is used as a component of these formulations. A blend of NaCMC grades is used to achieve desired properties. Differences between grades are defined by the degree of substitution (DS) of carboxymethyl groups onto the anhydroglucose repeat units of the cellulose chain. Higher DS grades (>1.0) are used to impart a high rate of absorption, while lower to moderate DS grades (<1.0) are included for their long term cohesiveness. One further requirement of the formulation is that its response to moisture must not compromise the performance of the device for which the adhesive was intended. As hydrocolloid adhesives absorb moisture they begin to swell. The hydrating hydrocolloid powders transform the rubbery elastomer phase into a more gelatinous composition that appears to grow. Typically this growth occurs in the direction of a free surface. For wound dressings the growth is into the wound. For ostomy skin barriers the growth is toward the stoma, and is called turtlenecking for its resemblance to the collar on a turtleneck sweater.

In some cases turtlenecking is desirable, as it has been reported to provide an effective seal around the stoma itself and prevents leakage of stomal effluent and undermining of the skin barrier. However, excessive amounts of turtlenecking can have the opposite effect, causing effluent to be diverted from the pouch and against the skin. Excessive turtlenecking in hydrocolloid adhesives can be avoided by limiting elongation of the elastomeric phase during hydration. Key factors in attaining acceptable turtlenecking are the choice of elastomer, the balance of absorption rate and capacity within the limits described above, the balance of cohesive and adhesive properties in the elastomer phase.

In contrast to denture adhesives, it has been discovered that use of higher DS CMC grades in combination with low to moderate DS grades reduces the rate of moisture absorption in hydrocolloid adhesives. In other words, as the average degree of substitution of the CMC in the formulation increases, the absorption rate of the hydrocolloid adhesive decreases. Further, overall absorption capacity increases. As a result, longer wearing times are possible because the influence of stomal effluent on adhesive integrity is reduced, though the film forming and water activated bonding capabilities of CMC are retained. Additionally, turtlenecking characteristics are favorably impacted.

Although it is possible to lower the absorption rate of the formulation by using different hydrocolloids having a weaker affinity for water, the impact on overall adhesive properties is not as favorable as with the use of higher DS CMC. Examples 34 and 35 below show how use of pregelatinized starch, a low absorbing hydrocolloid, reduces the absorption rate of the formulation. However, in contrast with the performance of high DS CMC, the overall absorption capacity of the adhesive is not increased. Further, the water activated bonding power of hydrated starch to human skin is not believed to be as effective as either pectin or especially CMC. Therefore, addition of a minor amount of starch, or other low absorbing hydrocolloids, may expand the useful range of this invention to the extent that the effectiveness of the preferred hydrocolloids in extending wear time is not compromised:

Addition of a small amount of a powdered cellulose, which is not a hydrocolloid but a water swellable powder, is also useful for reducing moisture absorption while still being of similar composition to NaCMC. A further benefit of adding powdered cellulose is that it improves cohesive strength of the overall formulation.

As a result of the fact that the EPR is can be readily blended with the other formulation components, a variety of conventional compounding processes are believed to be suitable for obtaining a homogeneous mixture. Potential mixing processes would include solvent blending, continuous or semi-continuous compounding, calendering or milling, and internal or external mixers. The preferred mixing process utilizes a high intensity batch mixer, which has been heated to between 250 and 330□F. The process proceeds by alternating additions of rubber or block copolymer with any of the powders and one of the low molecular weight components, which are mixed in groups until homogeneous. The entire process proceeds for as long as about 90 minutes or more. The low molecular weight components include any of the tackifiers and the plasticizer. The plasticizer used should be a low molecular weight polymer appropriately chosen to reduce the modulus of the formulation, promote flexibility and conformability of the adhesive, and be suitable for contact with the skin or mucosal tissue. Preferably petrolatum may be used.

Formulated adhesive mass may then be formed by any of various means into smooth sheets. The preferred forming methods are extrusion or compression. A useful thickness range for the adhesive is believed to be in the range between 0.005 inch and 0.25 inch. This adhesive sheet may be laminated with a wide variety of films, foams, non-woven or other fabrics, etc. and also to paper, some of which may have been coated with release agents to promote removal. The adhesive sheet may be cut into useful sizes, shapes and dimensions including discs, profiles, contours or other constructions of adhesive articles. Laminates may be added readily to the adhesive when it is maintained at elevated temperature. Preferred methods of lamination include a roll based lamination station or a compression type process. Cutting may be accomplished using several methods, the preferred ones being a rotary cutting die or a platen type cutting die.

### EXAMPLES

Table 2 provides a listing of 25 hydrocolloid adhesives pursuant to the present invention. Each of these compositions are blended to produce compositions with the characteristics listed in Table 2A within the usefulness ranges of Table 1.

Additional examples showing the utility of the invention are shown in Table 6.

Examples of the trade names for components of the compositions are the following:
ethylene propylene rubber - Vistalon 404, Exxon Chemical Co.;
styrenic block copolymer - Kraton D1107, Shell Chemical Co.;
tackifier (solid) - Piccotac 95, Hercules, Inc.;
tackifier (liquid) - Regalrez 1018, Hercules, Inc.;
NaCMC - Sodium Carboxymethyle cellulose FCC grade with
   DS7 = degree of substitution = 0.7
   DS12 = degree of substitution = 1.2
pectin - Pectin, USP 100;
powdered cellulose, FCC - Solka Floc 200 FCC, Fiber Sales & Development Corp.
Antioxidant - Irganox 1010, Ciba Geigy Corp.;
Plasticizer - white petrolatum, USP;
Cyclo-aliphatic tackifying resin - Relarez 1094, Hercules, Inc.;
Hydrogenated rosin ester - Pentalyn H., Hercules, Inc.;
Pregelatinized starch - Prejel PA5, Avebe, Inc.

Examples 1-4 and 17-20 of Tables 2 and 2A demonstrate acceptable performance within the usefulness ranges of Table 1. These examples represent a base adhesive which has no liquid tackifier, high DS DMC, or powdered cellulose but still achieves useful properties. Improvements to probe tack resulting from the use of low softening point tackifier are shown in Table 3. The effectiveness of high degree of substitution sodium carboxymethyl cellulose to reduce absorption is shown in Table 5. The ability of powdered cellulose to reduce absorption and increase strength is demonstrated in Table 4.

Measurements of probe tack were based on the method described in ASTM D2979 using an inverted probe machine. In this test, conducted at room temperature, the dwell time was 1 second, and the approach speed was 1 cm/second. Tensile measurements were based on ASTM test method D412 with the crosshead speed set to 200 mm/minute using a dumb bell shaped test specimen. The peak tensile strength is reported. Absorbency measurements were conducted based on the British Pharmacopoeia method wherein a flat adhesive specimen of known weight and area is exposed to 0.9% saline solution. The exposed adhesive is incubated at body temperature for 24 hours and re-weighed. The weight difference per area is reported.

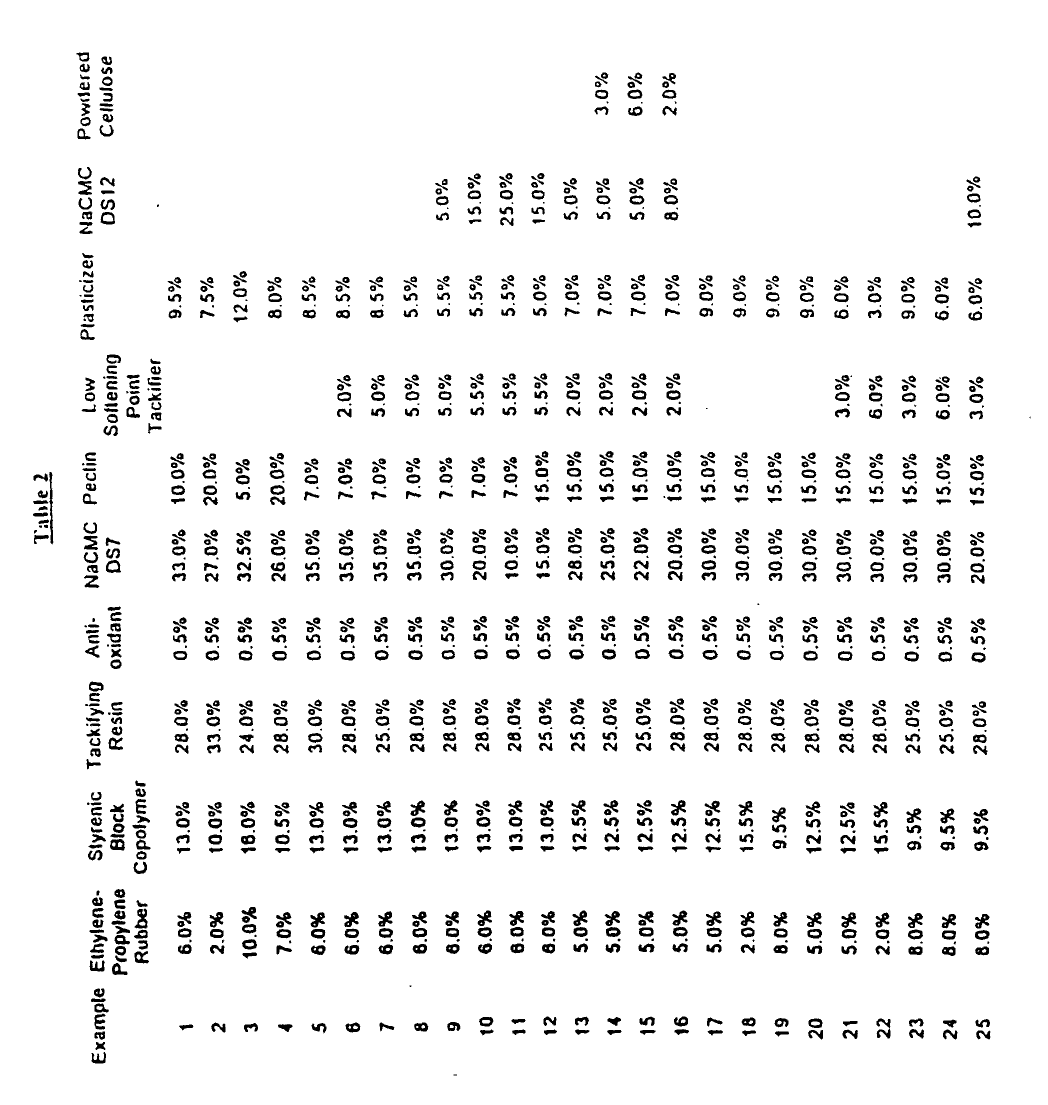

**Table 2A**

| Example | Probe Tack g tack force | Tensile Strength g/cm^2 (non-extruded) | 24 Hr Saline Absorption g/sq.m/24 hr (non-extruded) |
|---|---|---|---|
| 1 | 432 | 852 | 1937 |
| 2 | 411 | 2966 | 3459 |
| 3 | 470 | 860 | 1439 |
| 4 | 418 | 1465 | 2936 |
| 5 | 559 | 1690 | 2111 |
| 6 | 481 | 1451 | 2479 |
| 7 | 517 | 1637 | 2947 |
| 8 | 615 | 1690 | 3507 |
| 9 | 586 | 1587 | 2859 |
| 10 | 418 | 1574 | 2113 |
| 11 | 438 | 2065 | 1538 |
| 12 | 477 | 1757 | 2257 |
| 13 | 572 | 1032 | 3590 |
| 14 | 449 | 1919 | 3237 |
| 15 | 425 | 1830 | 2446 |
| 16 | 599 | 1312 | 1869 |
| 17 | 508 | 662 | 2200 |
| 18 | 426 | 1538 | 2705 |
| 19 | 529 | 824 | 3249 |
| 20 | 491 | 1205 | 2209 |
| 21 | 531 | 998 | 2843 |
| 22 | 420 | 2183 | 2861 |
| 23 | 394 | 587 | 2326 |
| 24 | 450 | 632 | 3107 |
| 25 | 597 | 873 | 2426 |

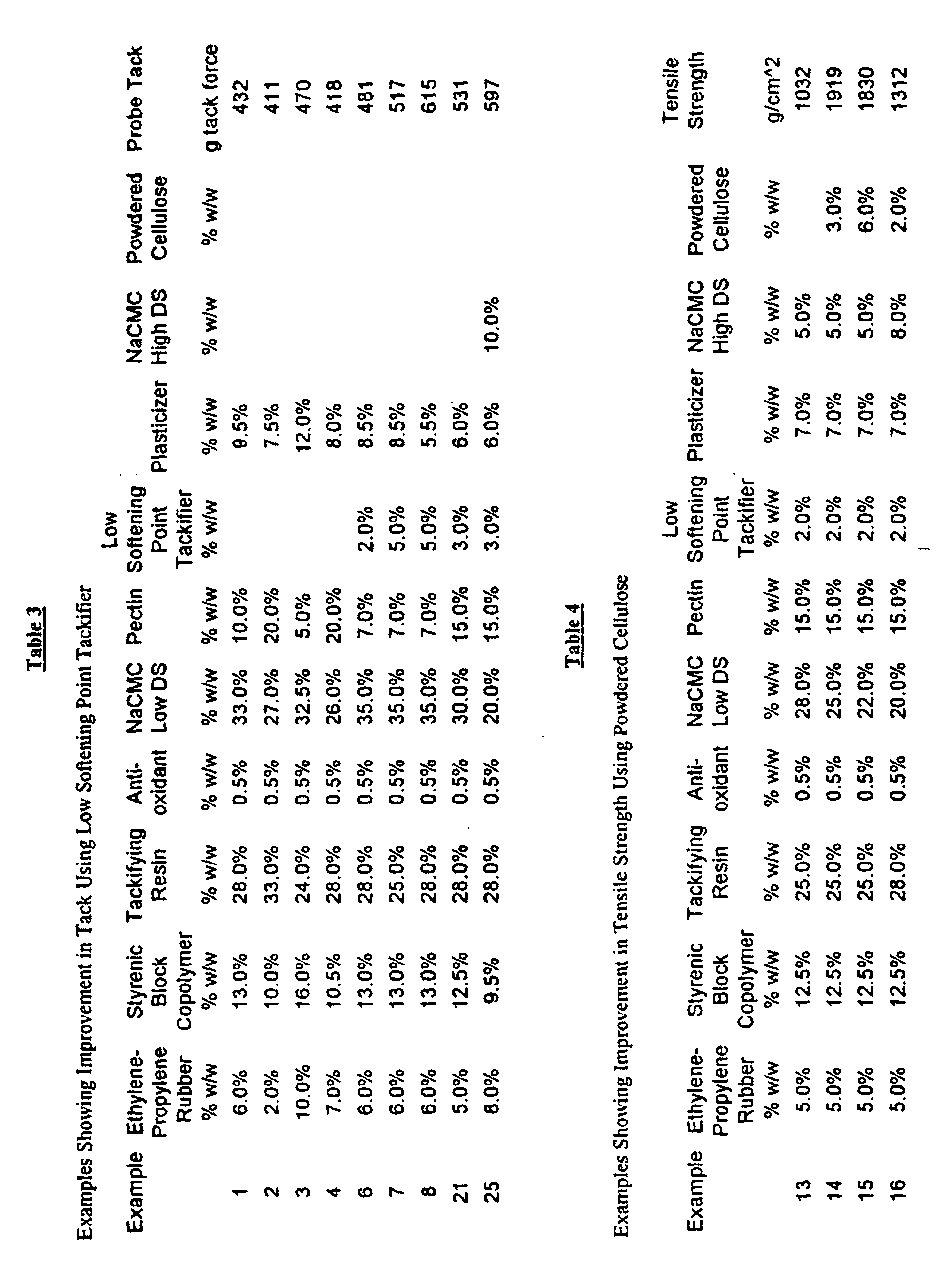

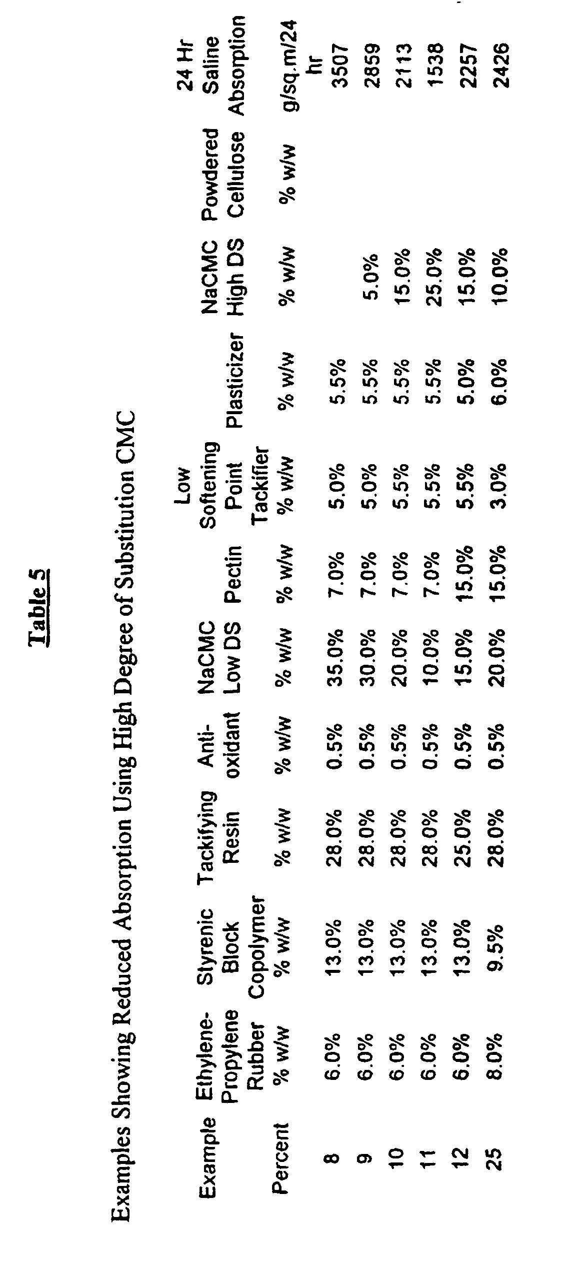

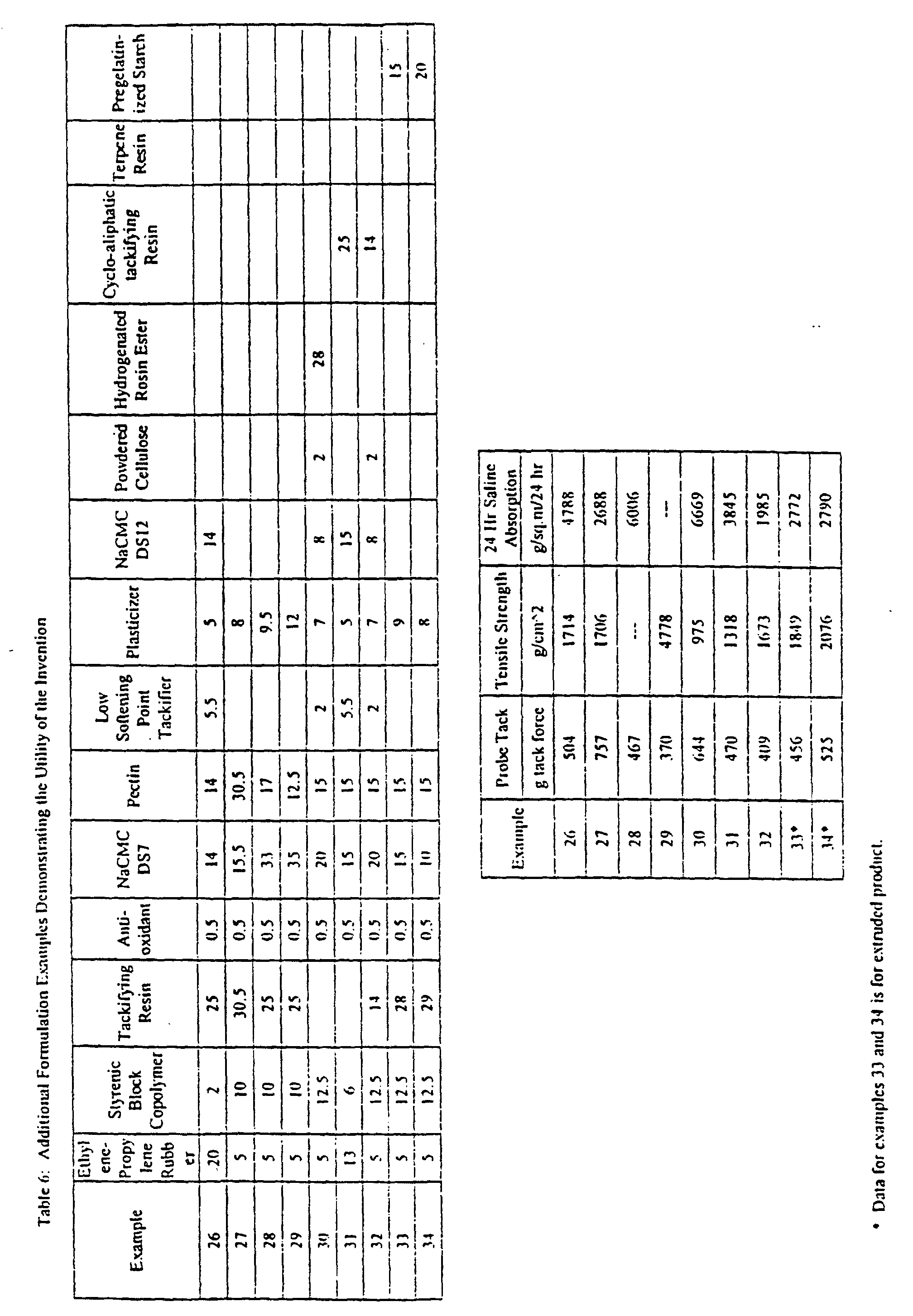

## Claims

1. A pressure sensitive hydrocolloid adhesive comprising the following composition by percentage weight:
a) from about 2% to about10% ethylene propylene rubber
b) from about 9.5% to about16% styrenic block copolymer
c) from about 24% to about33% tackifying resin
d) from 0% to about .5% anti-oxidant
e) from about 15% to about35% NaCMC (Low DS)
f) from about 5% to about20% pectin
g) from 0% to about 6% tackifier with low softening point
h) from about 3% to about12% plasticizer
i) from 0% to about 25% NaCMC (high DS)
j) from 0% to about 6% powdered cellulose
wherein the probe tack force in grams is in the range of 400-750.

2. The hydrocolloid adhesive of claim 1 wherein the ethylene propylene rubber has a broad molecular weight distribution.

3. The hydrocolloid adhesive of claim 1 wherein the ethylene propylene rubber is amorphous and random.

4. The hydrocolloid adhesive of claim 1 wherein the ethylene propylene rubber has an ethylene content of 50% or less.

5. The hydrocolloid adhesive of claim 1 wherein the probe tack force in grams is in the range of 500-650 grams.

6. The hydrocolloid adhesive of claim 1 wherein the saline absorbency in grams per square meter for a 24 hour period is in the range of 1250 - 5000.

7. The hydrocolloid adhesive of claim 1 wherein the saline absorbency in grams per square meter for a 24 hour period is in the range of 2000 - 3500 when extruded.

8. The hydrocolloid adhesive of claim 1 wherein the saline absorbency in grams per square meter for a 24 hour period is in the range of 1500 - 2500 when non-extruded.

9. The hydrocolloid adhesive of claim 1 wherein the non-extruded tensile strength in grams per square centimeter is in the range of 800 - 1500.

10. The hydrocolloid adhesive of claim 1 wherein the tensile strength in grams per square centimeter is in the range of 500 - 3500.

11. The hydrocolloid adhesive composition of claim 1 wherein the extruded tensile strength is in the range of 1500 - 2500 grams per square centimeter.

12. The hydrocolloid adhesive of claim 1 wherein the probe tack is between about 300 to about 750 grams, force.

13. The hydrocolloid adhesive of claim 1 wherein the absorption of saline at 37°C is between about 500 and about 5,000 grams per square meter per day.

14. The hydrocolloid adhesive of claim 1 wherein the tensile strength is between about 500 and 3,500 grams per square centimeter.

15. The hydrocolloid adhesive of claim 1 wherein the tackifier softening point is below about 37°C.

16. A pressure sensitive hydrocolloid adhesive comprising the following composition by percentage weight:
a) from about 2% to about 20% ethylene propylene rubber.
b) zfrom about 2% to about 16% styrenic block copolymer.
c) from about 14% - about 33% tackifying resin selected from the group of aliphatic, cyclo-aliphatic, mixed aliphatic-aromatic, hydrocarbon, pure monomer, rosins, gums and their esters and derivatives, or terpene or polyterpene resins.
d) from 0% to about 0.5% anti-oxidant.
e) from about 10% to about 35% NaCMC with degree of substitution below 1.0.
f) from 0% to about 30.5% pectin.
g) from about 3% to about 12% plasticizer.
h) from 0% to about 6% tackifier with softening point below about 37°C.
i) from 0% to about 25% NaCMC with degree of substitution above 1.0.
j) from 0% to about 6% powdered cellulose.

17. The hydrocolloid adhesive of claims 16 wherein the probe tack is between about 300 to about 750 grams, force.

18. The hydrocolloid adhesive of claims 16 wherein the absorption of saline at 37°C is between about 500 and about 5,000 grams per square meter per day.

19. The hydrocolloid adhesive of claims 16 wherein the tensile strength is between about 500 and 3,500 grams per square centimeter.

20. A pressure sensitive hydrocolloid adhesive comprising the following composition by percentage weight:
a) from about 11.5% to about 36% of a hydrocolloid blend of ethylene propylene rubber and styrenic block copolymer.
b) from about 24% to about 39% tackifying resin
c) from 0% to about 0.5% anti-oxidant.
d) from about 20% to about 52% absorbent powder selected from the group NaCMC, Pectin, powdered cellulose, and pregelatinized starch, optionally including minor amounts of powdered fillers, fibers, absorbents, or super absorbents.
e) from about 3% to about 12% plasticizer.
f) from 0% to about 6% tackifier with softening point below about 37°C.
g) from 0% to about 25% NaCMC with degree of substitution above 1.0.
h) from 0% to about 6% powdered cellulose.

21. The hydrocolloid adhesive of claim 20 wherein the probe tack is between about 300 to about 750 grams, force.

22. The hydrocolloid adhesive of claim 20 wherein the absorption of saline at 37°C is between about 500 and about 5,000 grams per square meter per day.

23. The hydrocolloid adhesive of claim 20 wherein the tensile strength is between about 500 and 3,500 grams per square centimeter.

## Patentansprüche

1. Druckempfindlicher hydrokolloider Klebstoff mit der folgenden Zusammensetzung in Masseprozent:
a) von etwa 2 % bis etwa 10 % Ethylen-Propylen-Kautschuk,
b) von etwa 9,5 % bis etwa 16 % Styrenblockcopolymer,
c) von etwa 24 % bis etwa 33 % klebrigmachendes Harz,
d) von 0 % bis etwa 0,5 % Antioxidationsmittel,
e) von etwa 15 % bis etwa 35 % Natriumcarboxymethylcellulose (NaCMC) (niedriger Substitutionsgrad),
f) von etwa 5 % bis etwa 20 % Pektin,
g) von 0 % bis etwa 6 % Klebrigmacher mit einem niedrigen Erweichungspunkt,
h) von etwa 3 % bis etwa 12 % Weichmacher,
i) von 0 % bis etwa 25 % NaCMC (hoher Substitutionsgrad),
j) von 0 % bis etwa 6 % pulverisierte Cellulose,
wobei die Klebkraft der Probe in dem Bereich von 400 - 750 Pond liegt.

2. Hydrokolloider Klebstoff nach Anspruch 1, **dadurch gekennzeichnet, dass** der Ethylen-Propylen-Kautschuk eine breite Verteilung der relativen Molekülmassen hat.

3. Hydrokolloider Klebstoff nach Anspruch 1, **dadurch gekennzeichnet, dass** der Ethylen-Propylen-Kautschuk amorph und statistisch ist.

4. Hydrokolloider Klebstoff nach Anspruch 1, **dadurch gekennzeichnet, dass** der Ethylen-Propylen-Kautschuk einen Ethylengehalt von 50 % oder weniger hat.

5. Hydrokolloider Klebstoff nach Anspruch 1, **dadurch gekennzeichnet, dass** die Klebkraft der Probe in dem Bereich von 500 - 650 Pond liegt.

6. Hydrokolloider Klebstoff nach Anspruch 1, **dadurch gekennzeichnet, dass** das Absorptionsvermögen der Salzlösung für einen Zeitraum von 24 Stunden in dem Bereich von 1250 - 5000 Gramm je Quadratmeter liegt.

7. Hydrokolloider Klebstoff nach Anspruch 1, **dadurch gekennzeichnet, dass** das Absorptionsvermögen der Salzlösung für einen Zeitraum von 24 Stunden in dem Bereich von 2000 - 3500 Gramm je Quadratmeter liegt, wenn er extrudiert ist.

8. Hydrokolloider Klebstoff nach Anspruch 1, **dadurch gekennzeichnet, dass** das Absorptionsvermögen der Salzlösung für einen Zeitraum von 24 Stunden in dem Bereich von 1500 - 2500 Gramm je Quadratmeter liegt, wenn er nichtextrudiert ist.

9. Hydrokolloider Klebstoff nach Anspruch 1, **dadurch gekennzeichnet, dass** die Zugfestigkeit des nichtextrudierten Klebstoffs in dem Bereich von 800 - 1500 Pond je Quadratzentimeter liegt.

10. Hydrokolloider Klebstoff nach Anspruch 1, **dadurch gekennzeichnet, dass** die Zugfestigkeit in dem Bereich von 500 - 3500 Pond je Quadratzentimeter liegt.

11. Hydrokolloide Klebstoff-Zusammensetzung nach Anspruch 1, **dadurch gekennzeichnet, dass** die Zugfestigkeit des extrudierten Klebstoffs in dem Bereich von 1500 - 2500 Pond je Quadratzentimeter liegt.

12. Hydrokolloider Klebstoff nach Anspruch 1, **dadurch gekennzeichnet, dass** die Klebkraft der Probe zwischen etwa 300 und etwa 750 Pond liegt.

13. Hydrokolloider Klebstoff nach Anspruch 1, **dadurch gekennzeichnet, dass** das Absorptionsvermögen der Salzlösung bei 37 °C zwischen etwa 500 und etwa 5000 Pond je Quadratmeter je Tag liegt.

14. Hydrokolloider Klebstoff nach Anspruch 1, **dadurch gekennzeichnet, dass** die Zugfestigkeit zwischen etwa 500 und 3500 Pond je Quadratzentimeter liegt.

15. Hydrokolloider Klebstoff nach Anspruch 1, **dadurch gekennzeichnet, dass** der Erweichungspunkt des Klebrigmachers unter etwa 37 °C liegt.

16. Druckempfindlicher hydrokolloider Klebstoff mit der folgenden Zusammensetzung in Masseprozent:
a) von etwa 2 % bis etwa 20 % Ethylen-Propylen-Kautschuk,
b) von etwa 2 % bis etwa 16 % Styrenblockcopolymer,
c) von etwa 14 % bis etwa 33 % klebrigmachendes Harz, das aus der Gruppe Aliphaten, Cycloaliphaten, gemischte Aliphaten-Aromaten, Kohlenwasserstoffe, reine Monomere, Terpentinharze, Kautschuke und deren Ester und Derivate, oder Terpenoder Polyterpenharze gewählt ist,
d) von 0 % bis etwa 0,5 % Antioxidationsmittel,
e) von etwa 10 % bis etwa 35 % NaCMC mit einem Substitutionsgrad unter 1,0,
f) von 0 % bis etwa 30,5 % Pektin,
g) von etwa 3 % bis etwa 12 % Weichmacher,
h) von 0 % bis etwa 6"% Klebrigmacher mit einem Erweichungspunkt unter etwa 37 °C,
i) von 0 % bis etwa 25 % NaCMC mit einem Substitutionsgrad über 1,0,
j) von 0 % bis etwa 6 % pulverisierte Cellulose.

17. Hydrokolloider Klebstoff nach Anspruch 16, **dadurch gekennzeichnet, dass** die Klebkraft der Probe zwischen etwa 300 und etwa 750 Pond liegt.

18. Hydrokolloider Klebstoff nach Anspruch 16, **dadurch gekennzeichnet, dass** das Absorptionsvermögen der Salzlösung bei 37 °C zwischen etwa 500 und etwa 5000 Gramm je Quadratmeter je Tag liegt.

19. Hydrokolloider Klebstoff nach Anspruch 16, **dadurch gekennzeichnet, dass** die Zugfestigkeit zwischen etwa 500 und 3500 Pond je Quadratzentimeter liegt.

20. Druckempfindlicher hydrokolloider Klebstoff mit der folgenden Zusammensetzung in Masseprozent:
a) von etwa 11,5 % bis etwa 36 % hydrokolloides Gemisch aus Ethylen-Propylen-Kautschuk und Styrenblockcopolymer,
b) von etwa 24 % bis etwa 39 % klebrigmachendes Harz,
c) von 0 % bis etwa 0,5 % Antioxidationsmittel,
d) von etwa 20 % bis etwa 52 % absorptionsfähiges Pulver, das aus der Gruppe NaCMC, Pektin, pulverisierte Cellulose und vorgelatinierte Stärke, die wahlweise geringe Mengen von pulverisierten Füllstoffen, Fasern, Absorbenzien oder Superabsorbenzien enthalten, gewählt ist,
e) von etwa 3 % bis etwa 12 % Weichmacher,
f) von 0 % bis etwa 6 % Klebrigmacher mit einem Erweichungspunkt unter etwa 37 °C,
g) von 0 % bis etwa 25 % NaCMC mit einem Substitutionsgrad über 1,0,
h) von 0 % bis etwa 6 % pulverisierte Cellulose.

21. Hydrokolloider Klebstoff nach Anspruch 20, **dadurch gekennzeichnet, dass** die Klebkraft der Probe zwischen etwa 300 und etwa 750 Pond liegt.

22. Hydrokolloider Klebstoff nach Anspruch 20, **dadurch gekennzeichnet, dass** das Absorptionsvermögen der Salzlösung bei 37 °C zwischen etwa 500 und etwa 5000 Gramm je Quadratmeter je Tag liegt.

23. Hydrokolloider Klebstoff nach Anspruch 20, **dadurch gekennzeichnet, dass** die Zugfestigkeit zwischen etwa 500 und 3500 Pond je Quadratzentimeter liegt.

## Revendications

1. Adhésif hydrocolloïdal sensible à la pression comprenant la composition suivante en pourcentage en poids :
a) d'environ 2 % à environ 10 % de caoutchouc d'éthylène-propylène
b) d'environ 9,5 % à environ 16 % d'un copolymère séquencé styrénique
c) d'environ 24 % à environ 33 % de résine collante
d) de 0% à environ 0,5 % d'anti-oxydant
e) d'environ 15 % à environ 35 % de NaCMC (faible degré de substitution DS)
f) d'environ 5 % à environ 20 % de pectine
g) d'environ 0 % à environ 6 % d'agent de collage ayant un faible point de ramollissement
h) d'environ 3 % à environ 12 % de plastifiant
i) de 0 % à environ 25 % de NaCMC (degré élevé de substitution DS)
j) de 0 % à environ 6 % de cellulose en poudre
dans lequel la force de collage à la sonde en grammes est dans la plage de 400 à 750.

2. Adhésif hydrocolloïdal selon la revendication 1, dans lequel le caoutchouc d'éthylène-propylène présente une distribution de masse moléculaire large.

3. Adhésif hydrocolloïdal selon la revendication 1, dans lequel le caoutchouc d'éthylène-propylène est amorphe et aléatoire.

4. Adhésif hydrocolloïdal selon la revendication 1, dans lequel le caoutchouc d'éthylène-propylène présente une teneur en éthylène de 50 % ou moins.

5. Adhésif hydrocolloïdal selon la revendication 1, dans lequel la force de collage à la sonde en grammes est dans la plage de 500 à 650 grammes.

6. Adhésif hydrocolloïdal selon la revendication 1, dans lequel la capacité d'absorption de solution saline en grammes par mètre carré pendant une période de 24 heures est dans la plage de 1 250 à 5 000.

7. Adhésif hydrocolloïdal selon la revendication 1, dans lequel la capacité d'absorption de solution saline en grammes par mètre carré pendant une période de 24 heures est dans la plage de 2 000 à 3 500, lorsqu'il est extrudé.

8. Adhésif hydrocolloïdal selon la revendication 1, dans lequel la capacité d'absorption de solution saline en grammes par mètre carré pendant une période de 24 heures est dans la plage de 1 500 à 2 500, lorsqu'il n'est pas extrudé.

9. Adhésif hydrocolloïdal selon la revendication 1, dans lequel la résistance à la traction à l'état non extrudé en grammes par centimètre carré est dans la plage de 800 à 1 500.

10. Adhésif hydrocolloïdal selon la revendication 1, dans lequel la résistance à la traction en grammes par centimètre carré est dans la plage de 500 à 3 500.

11. Composition d'adhésif hydrocolloïdal selon la revendication 1, dans lequel la résistance à la traction à l'état extrudé est dans la plage de 1 500 à 2 500 grammes par centimètre carré.

12. Adhésif hydrocolloïdal selon la revendication 1, dans lequel le collage à la sonde est entre environ 300 et environ 750 grammes force.

13. Adhésif hydrocolloïdal selon la revendication 1, dans lequel l'absorption de solution saline à 37 °C est entre environ 500 et environ 5 000 grammes par mètre carré par jour.

14. Adhésif hydrocolloïdal selon la revendication 1, dans lequel la résistance à la traction est entre environ 500 et 3 500 grammes par centimètre carré.

15. Adhésif hydrocolloïdal selon la revendication 1, dans lequel le point de ramollissement de l'agent de collage est en dessous d'environ 37 °C.

16. Adhésif hydrocolloïdal sensible à la pression comprenant la composition suivante en pourcentage en poids :
a) d'environ 2 % à environ 20 % de caoutchouc d'éthylène-propylène.
b) d'environ 2 % à environ 16 % d'un copolymère séquencé styrénique.
c) d'environ 14 % à environ 33 % de résine collante sélectionnée parmi le groupe constitué d'un hydrocarbure aliphatique, cyclo-aliphatique, aliphatique-aromatique mixte, d'un monomère pur, de rosines, de gommes et de leurs esters et leurs dérivés, ou de résines de terpène ou de polyterpène.
d) de 0 % à environ 0,5 % d'anti-oxydant.
e) d'environ 10 % à environ 35 % de NaCMC avec un degré de substitution en dessous de 1,0.
f) de 0 % à environ 30,5 % de pectine.
g) d'environ 3 % à environ 12 % de plastifiant.
h) de 0 % à environ 6 % d'agent de collage avec un point de ramollissement en dessous d'environ 37 °C.
i) de 0 % à environ 25 % de NaCMC avec un degré de substitution au-dessus de 1,0.
j) de 0 % à environ 6 % de cellulose en poudre.

17. Adhésif hydrocolloïdal selon la revendication 16, dans lequel le collage à la sonde est entre environ 300 et environ 750 grammes force.

18. Adhésif hydrocolloïdal selon la revendication 16, dans lequel l'absorption de solution saline à 37 °C est entre environ 500 et environ 5 000 grammes par mètre carré par jour.

19. Adhésif hydrocolloïdal selon la revendication 16, dans lequel la résistance à la traction est entre environ 500 et 3 500 grammes par centimètre carré.

20. Adhésif hydrocolloïdal sensible à la pression comprenant la composition suivante en pourcentage en poids :
a) d'environ 11,5 % à environ 36 % d'un mélange d'hydrocolloïdal de caoutchouc d'éthylène-propylène et d'un copolymère séquencé styrénique.
b) d'environ 24 % à environ 39 % de résine collante.
c) de 0 % à environ 0,5 % d'anti-oxydant.
d) d'environ 20 % à environ 52 % de poudre d'absorbant sélectionnée parmi le groupe du NaCMC, de la pectine, de la cellulose en poudre, et de l'amidon pré-gélifié, comprenant optionnellement des proportions mineures de charges en poudre, de fibres, d'absorbants, ou de super-absorbants.
e) d'environ 3 % à environ 12 % de plastifiant.
f) de 0 % à environ 6 % d'agent de collage avec un point de ramollissement en dessous d'environ 37 °C.
g) de 0 % à environ 25 % de NaCMC avec un degré de substitution au-dessus de 1,0.
h) de 0 % à environ 6 % de cellulose en poudre.

21. Adhésif hydrocolloïdal selon la revendication 20, dans lequel le collage à la sonde est entre environ 300 et environ 750 grammes force.

22. Adhésif hydrocolloïdal selon la revendication 20, dans lequel l'absorption de solution saline à 37 °C est entre environ 500 et environ 5 000 grammes par mètre carré par jour.

23. Adhésif hydrocolloïdal selon la revendication 20, dans lequel la résistance à la traction est entre environ 500 et 3 500 grammes par centimètre carré.
